# EUROPÄISCHE PATENTSCHRIFT

(11) **EP 3 500 152 B1**
(45) Veröffentlichungstag und Bekanntmachung des Hinweises auf die Patenterteilung: **18.10.2023**
(21) Anmeldenummer: 18769945.9
(22) Anmeldetag: 24.08.2018
(51) Int. Cl.: A61B 1/012, A61B 90/00, A61B 34/20, A61B 1/00, A61B 17/34

(54) **DETEKTIONSSYSTEM ZUR AUTOMATISCHEN DETEKTION CHIRURGISCHER INSTRUMENTE**
DETECTION SYSTEM FOR AUTOMATICALLY DETECTING SURGICAL INSTRUMENTS
SYSTÈME DE DÉTECTION POUR LA DÉTECTION AUTOMATIQUE D'INSTRUMENTS CHIRURGICAUX

(30) Priorität: 29.08.2017 DE 102017008148
(43) Veröffentlichungstag der Anmeldung: 26.06.2019
(73) Patentinhaber: Joimax GmbH, 76227 Karlsruhe (DE)
(72) Erfinder: RIES, Wolfgang, 76351 Linkenheim (DE); STEEGMÜLLER, Rainer, 70839 Gerlingen (DE); RIES, Maximilian, 76131 Karslruhe (DE)
(74) Vertreter: Lichti - Patentanwälte Partnerschaft mbB
(86) Internationale Anmeldenummer: PCT/EP2018/000413
(87) Internationale Veröffentlichungsnummer: WO 2019/042579

(56) Entgegenhaltungen:
- EP-A2- 1 442 715
- AU-B2- 2013 251 245
- US-A- 5 318 025
- US-A- 6 059 718
- US-A1- 2014 051 993

## Beschreibung

Die Erfindung wird in Anspruch 1 definiert und betrifft ein automatisches Detektionssystem zum automatischen Detektieren von chirurgischen Instrumenten und eine ein solches System aufweisende intraoperative Navigationsvorrichtung.

Minimal-invasive Operationen werden heute bereits mittels navigationsgestützter Operationsverfahren durchgeführt. Es werden hierzu unterschiedliche Navigationssysteme eingesetzt. Es kommen aktive und passive Systeme zum Einsatz. Bei aktiven Systemen ist ein in den Körper eines Patienten eingebrachtes Teil, wie ein Instrument oder chirurgisches Werkzeug mit einem Sender versehen, über den sich extern die Position des Instruments oder Werkzeugs, insbesondere des distalen am Eingriffsort befindlichen Endes bestimmen lässt.

Bei passiven Systemen sind neben optischen Systemen zur Positionierung eines chirurgischen Instruments, insbesondere von dessen distalem Ende auch elektromagnetische Systeme bekannt. Bei der elektromagnetischen Navigation wird durch einen Feldgenerator ein inhomogenes elektromagnetisches Feld erzeugt, welches über einen oder mehrere Sensoren erfasst wird, wodurch wiederum die Position und Ausrichtung des Instruments oder chirurgischen Werkzeugs, insbesondere dessen distales Ende, direkt oder indirekt erfasst werden kann. Direkte Erfassung des distalen Endes eines chirurgischen Teils beinhaltet die Anordnung des Sensors am distalen Ende des Teils selbst; indirekte Erfassung beinhaltet die feste starre Anbringung des Sensors in einer definierten Stelle, insbesondere Axialposition, an dem chirurgischen Teil. Aufgrund des gemessenen Sensorsignals können Rückschluss auf die Position und gegebenenfalls Ausrichtung des distalen Endes gesehen werden. Bei der passiven Navigation hat sich insbesondere die elektromagnetische Navigation bewährt, bei der ein elektromagnetisches Feld extern um den Operationsbereich, beispielsweise durch einen Erzeuger eines elektromagnetischen Feldes in einem Kissen, auf dem der Patient liegt, erzeugt wird. Im chirurgischen Instrument eingebaute spulenartige Sensoren ermöglichen die Ortung der Instrumente, woraufhin eine Darstellung in CT- oder MRT-Bildern erfolgen kann. Dieses Verfahren beinhaltet keine Strahlenbelastung und reduziert insgesamt so die Strahlenbelastung, auch durch einen reduzierten Röntgeneinsatz. Es wird weder die Bildqualität beeinträchtigt, noch können Sensoren, da es eben keine optischen Sensoren sind, verdeckt werden. Die Bewegungsfreiheit des Operateurs wird nicht eingeschränkt, wie es bei optischen Systemen der Fall ist.

Die US 6,059,718 zeigt Magnetfelderzeugungselemente oder Magnetfelddetektionselemente, die in einem elastischen Einführteil, das in einen Gegenstand eingeführt wird, durch ein isolierendes Material fixiert werden kann, wobei sichergestellt wird, dass die Formen der jeweiligen Magnetfelderzeugungselemente oder der jeweiligen Magnetfelddetektionselemente nicht verändert werden. Selbst wenn das Einfügeteil gebogen wird, ändert sich weder die Funktion der Magnetfelderzeugung noch die Funktion der Magnetfelderfassung. Magnetfelderfassungselemente oder Magnetfelderzeugungselemente, die an bekannten Positionen um das Objekt herum angeordnet sind, werden miteinander kombiniert, um die dreidimensionalen Positionen der Magnetfelderzeugungselemente oder der Magnetfelderfassungselemente innerhalb des Einführungsteils zu berechnen. Des Weiteren wird die Form des Einführungsteils geschätzt und ein dreidimensionales Bild, das der Form des Einführungsteils entspricht, erzeugt. Darüber hinaus wird ein dreidimensionales Bild auf eine Bildschirmoberfläche projiziert, um ein stereoskopisches Bild anzuzeigen, das der Form des Einlegeteils entspricht.

Die EP 1 442 715 A2 zeigt ein abstimmbares Implantat, ein System und ein Verfahren, die die Einstellung eines abstimmbaren Implantats in einem Patienten ermöglichen. Das abstimmbare Implantat umfasst einen Sicherungsmechanismus, um das Implantat im Patienten zu sichern, einen Betätigungsabschnitt, der es dem Implantat ermöglicht, sich zu bewegen und einen Einstellabschnitt, der die Einstellung des Implantats ermöglicht, nachdem das Implantat im Patienten positioniert wurde. Das Verfahren zum Einstellen des abstimmbaren Implantats umfasst das Analysieren des Betriebs des Implantats, das Bestimmen, ob irgendwelche Einstellungen erforderlich sind, und das Einstellen des Implantats, um die Leistung des Implantats zu verbessern. Das Implantatsystem umfasst sowohl das abstimmbare Implantat als auch ein telemetrisches System, das in der Lage ist, telemetrisch Daten von dem abstimmbaren Implantat zu empfangen, wobei die Daten verwendet werden, um festzustellen, ob eine Einstellung des abstimmbaren Implantats erforderlich ist. Das System umfasst auch eine Instrumentenbaugruppe, die zur Durchführung von Wirbelsäulenoperationen verwendet wird, wobei die Instrumentenbaugruppe eine Montageplattform und eine Spannvorrichtung umfasst.

Die US 5,318,025 A zeigt ein Verfolgungssystem, das magnetische Resonanzsignale verwendet, um die Position und Orientierung von mindestens einer Vorrichtung, wie z.B. einem Katheter, in einem Patienten zu überwachen. Die Vorrichtung verfügt über eine Vielzahl von Empfängerspulen, die für die im Patienten erzeugten Magnetresonanzsignale empfindlich sind. Diese Signale werden bei Vorhandensein von Magnetfeldgradienten erfasst und haben daher Frequenzen, die im Wesentlichen proportional zur Position der Spule entlang der Richtung des angelegten Gradienten sind. Die Signale werden als Reaktion auf sequentiell angelegte, zueinander orthogonale magnetische Gradienten erfasst, um die Position und Ausrichtung der Vorrichtung in mehreren Dimensionen zu bestimmen. Die vom Verfolgungssystem ermittelte Position und Ausrichtung des Geräts wird den unabhängig voneinander aufgenommenen medizinischen Diagnosebildern überlagert. Ein oder mehrere Geräte können gleichzeitig verfolgt werden.

Nach der AU 2013251245 B2 wird während der Erfassung von Ultraschalldaten in einem medizinischen Bildgebungsverfahren ein dreidimensionales Modell einer abzubildenden Struktur, z.B. eine elektroanatomische Karte, mit angezeigt und visuell markiert, um den Fortschritt der Datenerfassung anzuzeigen. Schnittebenen aufeinander folgender zweidimensionaler Bilder werden auf dem dreidimensionalen Modell als Linie oder farbiger Bereich markiert. Anhand dieser Anzeige kann der Bediener feststellen, in welchen Bereichen bereits ausreichend Daten erfasst wurden, und er wird auf Bereiche hingewiesen, in denen noch weitere Daten erfasst werden müssen. Es werden verschiedene Farbschemata verwendet, um die relative Vollständigkeit der Datenerfassung anzuzeigen.

Die US 2014/0051993 A1 zeigt ein Verfahren und Systeme zur Steuerung eines bildgebenden Verfahrens. Ein medizinisches Gerät wird in einen Patienten eingeführt und eine Position (z.B. Lage und/oder Ausrichtung) des medizinischen Geräts wird in dem Patienten relativ zu einer Referenzposition (z.B. eine zuvor erfasste Position des medizinischen Geräts oder eine Position innerhalb eines gewünschten Pfads) erfasst. Die Bildgebung des Patienten wird automatisch auf der Grundlage der erfassten relativen Position aktiviert, so dass der Patient nur zu relevanten Zeiten abgebildet wird. In diesem Fall wird die Bildgebung automatisch aktiviert, wenn das medizinische Gerät instabil ist und deaktiviert, wenn das medizinische Gerät stabil ist. Wenn beispielsweise Gewebe mit dem medizinischen Gerät abgetragen werden soll, kann das medizinische Personal über das Bild auf eine unbeabsichtigte Bewegung des medizinischen Geräts aufmerksam gemacht werden und eine etwaige Verlagerung des medizinischen Geräts von der Ablationsstelle korrigieren.

Der Erfindung liegt die Aufgabe zugrunde, ein Detektionssystem, eine Sensoreinheit und ein Navigationssystem zu schaffen, die eine automatische Detektion der Art eines chirurgischen Instruments und der Position im Patienten sowie eine entsprechende angepasste Anzeige auf einem Monitor für den Operateur bewirken.

Die Erfindung sieht im Hinblick auf die Lösung der genannten Aufgabe ein Detektionssystem vor mit mindestens zwei chirurgischen Instrumenten, die jeweils mindestens einen sich längs einer Längsachse des Instruments erstreckenden Hohlraum und einen proximalen Eingangsbereich zu dem Hohlraum aufweisen, wobei ein Winkel des proximalen Eingangsbereichs zur Längserstreckung des Hohlraums bei den mindestens zwei Instrumenten unterschiedlich ist, mit einer zwei elektromagnetische Sensoren aufweisenden in den Hohlraum einführbaren Sensoreinheit, dessen einer sich axial erstreckend im Hohlraum, dessen anderer im Eingangsbereich angeordnet ist, mit einem Feldgenerator zur Erzeugung eines elektromagnetischen Feldes, und mit einer Auswerteeinheit zur Auswertung von den Sensoren entsprechend ihrer Lage im Feld des Feldgenerators an die Auswerteeinheit übermittelter Signale. Im Rahmen der Erfindung weist das Detektionssystem mindestens zwei der folgenden Instrumente auf: Hohlnadel, Führungsstab, Zugangsnadel nach Yamshidi und/oder Endoskop eines solchen Detektionssystems. Weiterhin wird die Aufgabe durch eine intraoperative Navigationsvorrichtung mit einer Steuer- und Verarbeitungselektronik und mit mindestens einer mit dieser elektrisch verbindbaren Sensoreinheit der vorgenannten Art gelöst, bei dem die Steuer- und Verarbeitungselektronik eine Detektionseinheit, eine Navigationseinheit und ein Kamerasystem aufweist.

Typische Operations-Instrumente oder -Geräte sind solche, wie eine rotationssymmetrische Hohlnadel, ein Führungsstab, eine Zugangsnadel, insbesondere nach Yamshidi, die distal zum Operationsort hin geführt werden oder aber Endoskope mit einem abgewinkelten Einlasseinsatz, wie einem Spülanschluss. Die erfindungsgemäße Sensoreinheit ist bei Anordnung in der erstgenannten gestreckten Hohlnadel ebenfalls gestreckt, wodurch die beiden Sensoren axial hintereinander angeordnet und fluchtend ausgerichtet sind, also der Winkel zwischen ihnen Null ist. Demgegenüber befindet sich bei Anordnung der entsprechenden Sensoreinheit in einem Endoskop der distale Sensor am distalen Ende des lediglich axialen Schafts des Endoskops, während der proximale Sensor in einem Einlassansatz des Endoskops angeordnet ist und damit zur Achse des Schafts des Endoskops einen bestimmten Winkel einschließt und daher auch zu dem am distalen Ende vorgesehenen Sensor. Damit besteht ein Relativwinkel zwischen beiden Sensoren.

Der entsprechende abgewinkelte Eingangsbereich mit definierbarem abgewinkelten Einlass eines Endoskops und voneinander abweichenden anderen Winkeln können andere Instrumente, wie Führungsstab, Zugangsnadeln oder dergleichen aufweisen, wodurch die Winkelausrichtung ihrer befindlichen Sensoren von einer flexiblen Sensoreinheit bestimmt wird.

Die vorgenannten Unterschiede der Relativausrichtung der Sensoren können durch eine Auswerteeinheit eines elektronischen Systems detektiert werden und bilden daher die Voraussetzung zur Detektion der Art des chirurgischen Instruments oder Geräts, in dem die Sensoreinheit angeordnet ist. Dabei wird auch die Position des Instruments über die Sensoren bestimmt.

Die Detektion und Weiterverarbeitung der detektierten Information geschieht durch die genannte Auswerteeinheit, indem diese die Relativausrichtung der beiden Sensoren und der Position detektiert und eine Bildschirmanzeige auf einem Monitor eines intraoperativen Operationssystems für den Operateur derart steuert, dass das oder die für das jeweilige Instrument oder Gerät geeigneten Bilder angezeigt werden.

Mittels einer rotationssymmetrischen Hohlnadel wird ein Zugang zu einem Operationsgebiet im Körper eines Patienten geschaffen, so dass anschließend in an sich bekannter Weise andere Operationsinstrumente eingeführt werden können. Wird eine solche Nadel detektiert, durch die gegebenenfalls geeignete Arbeitsinstrumente eingeführt werden, werden automatisch neben jeweils einem sagittalen, koronalen und axialen CT-, MRT- oder Röntgen-Bild des Wirbelsäulenbereichs, insbesondere der Wirbelsäule und genauer des Orts der Wirbelsäule, bei dem sich das Operationsgebiet befindet, zusammen mit der diesem Bild durch orts- und orientierungsgerechte Überlagerung der Nadel bzw. Nadelspitze zusätzlich ein Bild entsprechend der Führungsrichtung der Hohlnadel und damit des einzusetzenden Arbeitsinstruments angezeigt, beispielsweise durch einen von der sonstigen Darstellung des Bildes deutlich abgehobenen Kreis als Projektion der Hohlnadelspitze auf den Operationsort als Orientierungshilfe zur richtigen Positionierung des Instruments.

Demgegenüber wird bei Erkennung eines Endoskops als chirurgischem Instrument oder Gerät neben den genannten sagittalen, koronalen und axialen Röntgenbildern mit überlagerter orts- und orientierungsgenauer Einblendung des Endoskopschaftes in das Röntgenbild zusätzlich automatisch das durch eine Kamera im Endoskop aufgenommene Bild des Operationsgebietes am Monitor des Operateurs angezeigt.

Entsprechendes gilt für weitere chirurgische Instrumente wie Führungsstäbe, Zugangsnadeln oder dergleichen.

Eine bevorzugte Ausgestaltung des erfindungsgemäßen Detektionssystems ist gekennzeichnet durch mindestens zwei der folgenden Instrumente: Hohlnadel, Führungsstab, Zugangsnadel nach Yamshidi und/oder Endoskop, wobei vorzugsweise der Führungsstab doppelt kanuliert ist. Eine bevorzugte Ausbildung des Detektionssystems ist gekennzeichnet durch mindestens vier unterschiedliche Instrumente.

In Weiterbildung ist vorgesehen, dass die Winkel der Eingangsbereiche zur Längserstreckung des Hohlraums zweier Instrumente sich um mindestens 5° voneinander unterscheiden, wobei die Eingangsbereiche der Instrumente vorzugsweise Winkel von 0°, 65° oder 35° zur Längserstreckung des Hohlraums des Instruments aufweisen.

Eine bevorzugte Weiterbildung sieht vor, dass die Sensoren einen festen Längsabstand zueinander aufweisen.

In einer Weiterbildung ist vorgesehen, dass die Sensoren Spulen aus elektrisch leitfähigem Draht sind, wobei insbesondere die Spulen aus Metall, insbesondere Kupfer oder Silber, bestehen.

In bevorzugten Weiterbildungen kann insbesondere vorgesehen sein, dass die Sensoreinheit einen die Sensoren ummantelnden flexiblen Schutzschlauch aufweist, wobei insbesondere der Schutzschlauch aus Kunststoff, insbesondere aus biokompatiblem, sterilisierbarem Kunststoff, wie Polyether besteht.

Zur exakten Festlegung der Sensoreinheit an dem chirurgischen Instrument oder Gerät wird ein Adapter, insbesondere ein Luer-Adapter, vorgesehen, der, wie bekannt, üblicherweise aus einem weiblichen und einem männlichen Adapterteil besteht. Demgemäß ist eine Sensoreinheit vorzugsweise dadurch ausgebildet, dass proximal des proximalen Sensors ein Adapterteil angeordnet ist. Dieses besteht vorzugsweise aus Kunststoff, wie sterilisierbarem, biokompatiblem Kunststoff, insbesondere Nylon.

Weitere bevorzugte Ausführungen sehen eine elektrische Verbindungsleitung der Sensoren am proximalen Ende, insbesondere zur Verbindung mit der Auswerteeinheit vor, wobei insbesondere die elektrische Verbindungsleitung von einem Schutzschlauch aus biokompatiblem, sterilisierbarem Kunststoff, insbesondere Silikon, umgeben ist und/oder dass der Schutzschlauch unmittelbar proximal des Adapterteils der Sensoreinheit von einem Knickschutz umgeben ist, wobei darüber hinaus in äußerst bevorzugter Ausgestaltung vorgesehen ist, dass der Knickschutz aus biokompatiblem, sterilisierbarem Kunststoff, insbesondere Silikon, umgeben ist.

Grundsätzlich ist dabei vorzugsweise vorgesehen, dass die Auswerteeinheit Teil einer Steuer- und Verarbeitungselektronik ist, wobei weiterhin ein Monitor vorgesehen sein kann. In äußerst bevorzugter Ausgestaltung ist die Ausbildung zur Darstellung mindestens zweier Instrumente, wie Hohlnadel, eines Führungsstabs, der insbesondere doppelt kanuliert ist, einer Zugangsnadel und/oder Endoskop, insbesondere des distalen Endes, Instrumente überlagert in einem Bild eines Wirbelsäulenbereichs eines Patienten vorgesehen.

Eine erfindungsgemäße intraoperative Navigationsvorrichtung weist eine Steuer- und Verarbeitungselektronik und ein erfindungsgemäßes Detektionssystem auf, wobei die Steuer- und Verarbeitungselektronik selbst eine Navigationseinheit aufweist, in die die Auswerteeinheit integriert ist.

Der Einsatz des erfindungsgemäßen Systems sieht vor, dass bei Detektion einer bestimmten relativen Winkelausrichtung der beiden Sensoren ein einer Winkelausrichtung entsprechendes Instrument erkannt und eine Darstellung des jeweiligen Instruments, insbesondere des distalen Endes, überlagert in einem Bild eines Wirbelsäulenbereichs eines Patienten bewirkt wird, wobei das Bild insbesondere ein CT-, MRT- oder Röntgen-Bild ist.

Ein Endoskop wird über die Steuereinheit des Kamerasystems gesteuert und gibt ein Bild an eine Verarbeitungseinheit des Kamerasystems. Dieses Bild wird an das Navigationssystem übertragen.

Das Navigationssystem bestehend aus verschiedenen funktionellen Softwarekomponenten, der Verarbeitungseinheit, der Steuereinheit und dem Feldgenerator, ist mit dem Kamerasystem und der Sensoreinheit sowie dem Monitor zur Anzeige verbunden. Die Sensoreinheit kann entweder zur Navigation eines rotationssymmetrischen Instruments oder einem Endoskop eingesetzt werden, indem diese entweder in den Schaft des Instruments oder in den Spülkanal des Endoskops eingeführt wird.

Der Feldgenerator erzeugt ein elektromagnetisches, inhomogenes Feld und wird über die Steuereinheit des Navigationssystems gesteuert. Die Signale der Sensorspulen des Drahtsensors werden über die Verarbeitungseinheit digitalisiert und über die funktionellen Softwarekomponenten ausgewertet. Die Funktionseinheit Instrumentenerkennung wertet die Signale der beiden Sensorspulen aus und erkennt anhand der Ausrichtung der beiden Spulen zueinander, ob die Sensoreinheit sich in einem Endoskop befindet oder nicht. Das Ergebnis gibt die Funktionseinheit Instrumentenerkennung an die Funktionseinheit Layout-Steuerung weiter. In Abhängigkeit ob die Navigationssoftware ein Endoskop erkennt oder nicht, wird über die Layout-Steuerung das entsprechende Layout auf dem Monitor dargestellt. Wird das Endoskop von der Software erkannt, wird das Videosignal über die Funktionseinheit in das Navigationssystem eingespeist und der Funktionseinheit für die Layout-Ausgabe zur Verfügung gestellt.

Wird kein Endoskop auf Basis der genau definierten Ausrichtung der beiden Sensorspulen zueinander über die entsprechende Funktionseinheit erkannt, wird ein rotationssymmetrisches Instrument und die entsprechende Layout-Darstellung ausgegeben.

Der Ablauf des Verfahrens ist vorzugsweise folgender:
Nach Starten der Navigationseinheit werden die Signale der Sensorik abgefragt. Sobald der Anwender die Sensoreinheit in das Arbeitsfeld des Feldgenerators bringt, kann ein Signal erkannt und gemessen werden. Ist das Sensorsignal ungleich dem Wert Null (Sensor im Arbeitsfeld), erfolgt eine Instrumentenerkennung. Hierfür wird die Ausrichtung der beiden Sensoren für die Sensorspulen ausgewertet. Sind dabei die beiden Sensoren zueinander entsprechend der Geometrie des Spülkanals eines Endoskops, d.h. mit Relativwinkel ungleich Null ausgerichtet (Abstand und Winkel), wird dies von der Software erkannt und entsprechend das Endoskop als navigiertes Instrument angezeigt. Des Weiteren wird eine Layout-Anpassung vorgenommen. Wurde das Endoskop als navigiertes Instrument erkannt, wird insbesondere das Signal der proximalen Spule genutzt um die Position des Endoskops im Arbeitsfeld zu messen und somit die Position relativ zum Bilddatensatz des Patienten anzuzeigen. Ist die Ausrichtung der beiden Sensoren ungleich der genau definierten einer in den Spülkanal des Endoskops eingeführten Sensoreinheit, wird ein rotationssymmetrisches Instrument (Hohlnadel) zusammen mit der entsprechenden Layout-Darstellung von der Navigationseinheit ausgegeben. Das Signal des distalen Drahtsensors wird zur Bestimmung der Instrumentenlage im Arbeitsfeld genutzt und entsprechend im Bilddatensatz des Patienten angezeigt.

Der Einsatz der erfindungsgemäßen Sensoren erfolgt vorzugsweise in einer Endoskop-Vorrichtung, die neben einem Endoskop eben die Sensoreinheit aufweist. Sie weist demgemäß eine Sensoreinheit mit mindestens zwei in Längsrichtung mit festem endlichem Abstand zu einander angeordneten Sensorspulen auf, die relativ in dem Endoskop zueinander unter einem endlichen Winkel ausgerichtet sind.

Dadurch, dass die beiden Sensoren unter einem endlichen Winkel relativ zueinander in der Endoskop-Vorrichtung angeordnet sind, wird erreicht, dass aufgrund der unterschiedlichen Anordnung die Orientierung der Endoskopvorrichtung im Magnetfeld einer Steuer- und Verarbeitungselektronik und damit auch im Raum präzise bestimmt werden kann.

In bevorzugter Ausgestaltung ist dabei vorgesehen, dass der erste Sensor parallel zur Mittelachse oder der Haupterstreckungsrichtung des Hohlraums ausgerichtet ist und der zweite Sensor unter einem endlichen Winkel zur Mittelachse ausgerichtet ist, wobei insbesondere der erste Sensor im Schaft des Endoskops angeordnet ist und die zweite Sensor in einem Ansatz des Einführkopfes. Damit kann durch das Sensorsignal der Sensoren im angelegten elektromagnetischen Feld auch deren Ort und aufgrund des festen Abstandes zum distalen Ende des Schaftes der Ort des distalen Endes des Schaftes im elektromagnetischen Feld und damit im Raum bestimmt werden.

In Weiterbildung ist vorgesehen, dass die die beiden Sensoren tragende Sensoreinheit sich durch einen Hohlraum der Endoskop-Vorrichtung erstreckt, wobei insbesondere die Sensoreinheit axial fest mit einem auf den Ansatz des Einführkopfes aufsetzbaren Adapter verbunden ist. Hierdurch wird der Ort des einen Sensors, insbesondere des - ersten - distalen Sensors am distalen Ende des Schaftes in der Endoskop-Vorrichtung genau festgelegt und damit aufgrund der Bestimmung des Ortes der ersten - distalen - Sensorspule durch eine Steuer- und Verarbeitungselektronik im Magnetfeld auch die exakte Bestimmung des genauen Ortes des distalen Arbeitsortes möglich.

Durch die feste Verbindung der die Sensorspulen tragenden Sensoreinheit mit einem vom Endoskop trennbaren Halter wird weiterhin erreicht, dass nach Positionierung der die den Sensor tragende Sensoreinheit entfernt und dass der durch diesen zur Positionierung belegte Hohlraum für andere Einsatzzwecke freigegeben werden kann. Weiter ist so die Sensoreinheit vom eigentlichen Endoskop trennbar und kann anderweitig eingesetzt werden. Dies ermöglicht auch eine einfachere Sterilisation.

Entsprechendes, wie vorstehend beschrieben, gilt auch für andere chirurgische Instrumente, wie Führungsstäbe, Zugangsnadeln oder dergleichen, worauf Vorstehendes in gleicher Weise anwendbar ist.

Weitere Merkmale und Vorteile der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt:
- Fig. 1: Eine erfindungsgemäße Sensoreinheit in gestrecktem Zustand;
- Fig. 2: die erfindungsgemäße Sensoreinheit der Fig. 1 im abgewickelten Zustand;
- Fig. 3: eine Hohlnadel mit einer gestreckten Sensoreinheit mit fluchtenden Sensoren;
- Fig. 4: die Anordnung einer erfindungsgemäßen Sensoreinheit in einem Endoskop zur Bildung einer Endoskopvorrichtung;
- Fig. 5: einen Längsschnitt eines doppelt kanulierten Führungsstabs mit aufgestecktem Adapter zur Aufnahme einer Sensoreinheit der Fig. 1, 2;
- Fig. 5a: den Führungsstab der Fig. 5 im Längsschnitt ohne Sensoreinheit;
- Fig. 5b: eine distale Stirnansicht des Führungsstabs der Fig. 5, 5a;
- Fig. 5c: eine proximale Stirnansicht des Führungsstabs der Fig. 5 mit aufgestecktem Adapter;
- Fig. 6: eine Punktionsnadel nach Yamshidi;
- Fig. 6a: einen Längsschnitt durch eine Punktionsnadel nach Yamshidi;
- Fig. 6b: einen teilweise geschnittenen Schnitt durch eine vollständige Yamshidi-Nadel, in der die Sensoren der Sensoreinheit (diese ohne Adapterteil) angedeutet sind;
- Fig. 7: ein Blockschaltbild des erfindungsgemäßen Navigationssystems;
- Fig. 8: ein Ablaufdiagramm des Verfahrens;
- Fig. 9: die mittels des erfindungsgemäßen Navigationssystems aufgrund der Ausrichtung der Sensoreinheit automatisch erzeugte Darstellung von chirurgischem Instrument in Überlagerung mit der Darstellung von Wirbelsäulenbereichen eines Patienten bei einer Hohlnadel; und
- Fig. 10: die mittels des erfindungsgemäßen Navigationssystems aufgrund der Ausrichtung der Sensoreinheit automatisch erzeugte Darstellung von chirurgischem Instrument in Überlagerung mit der Darstellung von Wirbelsäulenbereichen eines Patienten bei einem Endoskop; und

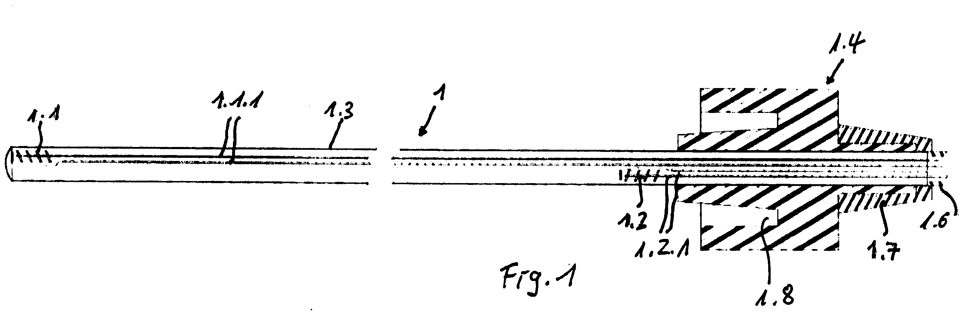

Die Fig. 1 zeigt eine erfindungsgemäße Sensoreinheit 1 als Teil eines erfindungsgemäßen Detektionssystems. Sie weist zwei mit festem Längsabstand zueinander angeordnete Sensoren 1.1, 1.2 auf, die als Spulen ausgebildet sind und im Folgenden so bezeichnet werden. Die Spulen 1.1, 1.2 haben einen festen Längsabstand. Sie sind in einem Schlauch 1.3 untergebracht. Jede Spule 1.1, 1.2 weist eine proximal anschließende Kontaktleitung 1.1.1, 1.2.1 auf. Spulen 1.1, 1.2 und Kontaktleitungen 1.1.1, 1.2.1 - jeweils mit Hin- und Rückleiter - bestehen aus leitfähigem Metall, vorzugsweise Kupfer. Sie sind voneinander elektrisch isoliert, beispielsweise durch Beschichtung mit Isolierlack. Proximal der proximalen Spule 1.2 ist ein männliches Luer-Adapterteil 1.4 vorgesehen. Dieses weist distal einen zylindrischen Ringraum 1.8 auf, mit dem es auf einen zylindrischen Mantel eines Einlasses eines entsprechenden chirurgischen Instruments oder dessen zugeordnetem Adapterteil definiert aufgebracht werden kann. Proximal desselben erstreckt sich ein Verbindungskabel 1.5 in dem die Kontaktleitungen 1.1.1, 1.2.1 der Spulen 1.1, 1.2 bis zu einem Anschlussstecker (nicht dargestellt) zum Anschluss einer Navigationsseinheit 3.1 geführt sind. Auch hier sind die Leitungen durch einen Mantel 1.6 ummantelt.

Unmittelbar proximal des Luer-Adapterteils 1.4 ist ein Knickschutz 1.7 vorgesehen, um ein Abknicken des Kabels 1.5 zu verhindern.

Schutzschlauch 1.3, Luer-Adapterteil 1.4, Knickschutz 1.7 und Mantel 1.6 bestehen vorzugsweise aus sterilisiertem Kunststoff, insbesondere biokompatiblem Kunststoff und zwar insbesondere Schlauch 1.3 aus Polyether, Luer-Adapterteil aus Nylon sowie Knickschutz 1.7 und Mantel 1.6 aus Silikon.

Werden die Spulen 1.1, 1.2 in ein inhomogenes elektromagnetisches Feld gebracht, wie dies durch die Steuer- und Verarbeitungselektronik 3 über einen Feldgenerator 2.1 erzeugt wird, so lässt sich mittels der Spulen sicher der Ort der Spulen sowie die Ausrichtung der Spule senkrecht zu ihrer Achse bestimmen, während eine Drehung um die Spulenachse selbst nicht erkannt werden kann. Ein solcher Sensor wird als 5-DOF-Sensor bezeichnet (DOF = Degree of Freedom).

Da es bei einer Hohlnadel, deren distales Ende sich am Operationsort befindet, auf die Kenntnis des Orts des distalen Endes der Hohlnadel (und damit auch eines separaten eingeführten Arbeitsinstruments) im Raum sowie die Ausrichtung der Hohlnadel (und des Arbeitsinstruments, nicht aber der Drehung der Hohlnadel und des Arbeitsinstruments um ihre Achse) ankommt, reicht ein solcher 5-DOF-Sensor für die Bestimmung von Ausrichtung des distalen Endes der Hohlnadel und des Arbeitswerkzeugs aus und kann durch einen Sensor, der bis zum distalen Ende der Hohlnadel eingeführt ist, im inhomogenen Feld bestimmt werden.

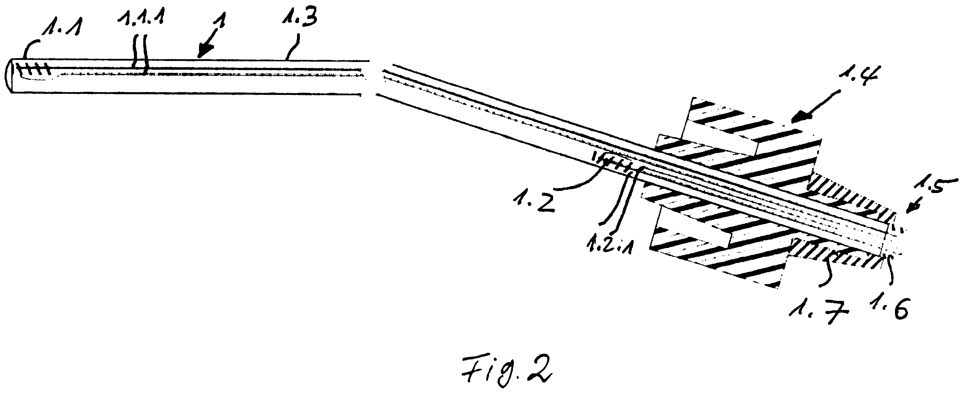

Gemäß Fig. 2 kann durch eine zweite entsprechende Spule 1.2, die mit bekanntem Längsabstand zur Spule 1.1 angeordnet ist, bei einem nicht-rotationssymmetrischen Gerät, wie einem Endoskop, bei Ausrichtung der beiden Spulen 1.1, 1.2 unter einem endlichen Winkel zueinander auch die Ausrichtung des fraglichen Instruments, wie eines Endoskops, bestimmt werden, wie dies bei der Darstellung der Fig. 2 der Fall ist.

Die Kombination der beiden Spulen 1.1, 1.2 und damit die Sensoreinheit 1 bilden damit insgesamt einen 6-DOF-Sensor.

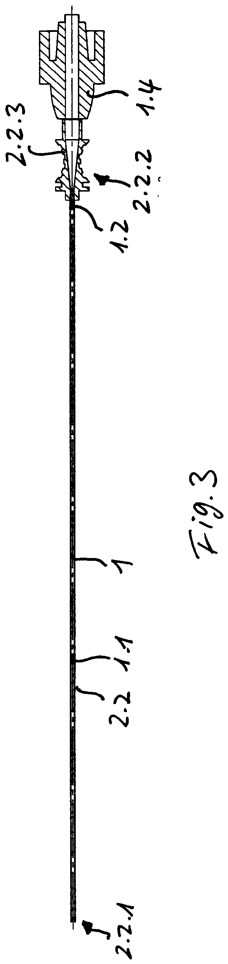

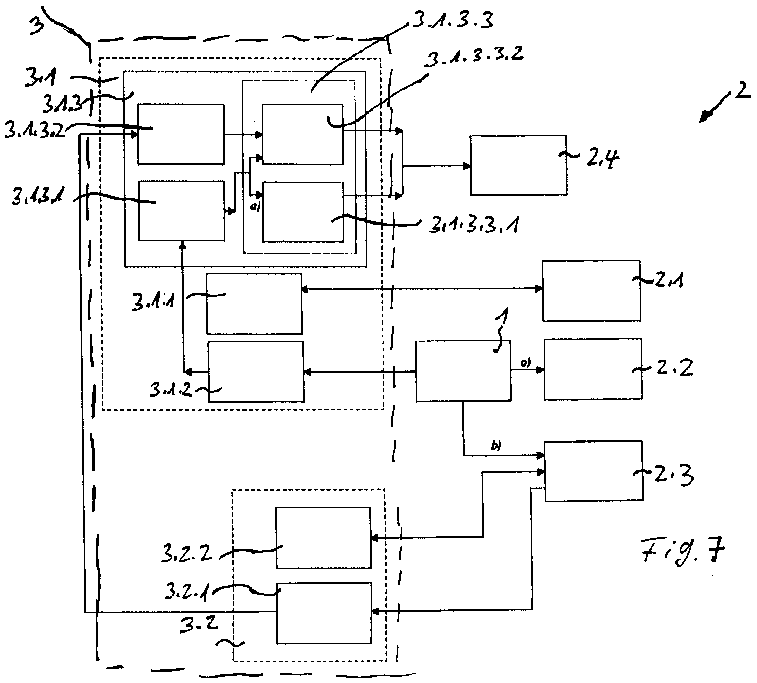

Die Fig. 3 zeigt eine Hohlnadel 2.2 mit einer in dieser befindlichen Sensoreinheit 1 als Teil des erfindungsgemäßen Detektionssystems. Die Hohlnadel ist an ihrem distalen Ende 2.2.1 abgeschrägt und trägt an ihrem proximalen Ende 2.2.2 ein Adapterteil 2.2.3. Die Sensoreinheit 1 weist, wie vorstehend beschrieben, einen distalen Sensor 1.1 und einen proximalen Sensor 1.2 auf. Darüber hinaus deckt sie am proximalen Ende das ebenfalls schon beschriebene Adapterteil 1.4. Die beiden Sensoren 1.1 und 1.2 sind gleich ausgerichtet und fluchtend zueinander angeordnet, so dass die Anordnung des Adapterteils in einer Hohlnadel 2.2 und damit diese aufgrund dieser Ausrichtung der beiden Sensoren 1.1, 1.2 der Sensoreinheit 1 erkannt werden kann, wie dies weiter unten unter Bezug auf die Fig. 7 bis Fig. 9 näher erläutert wird.

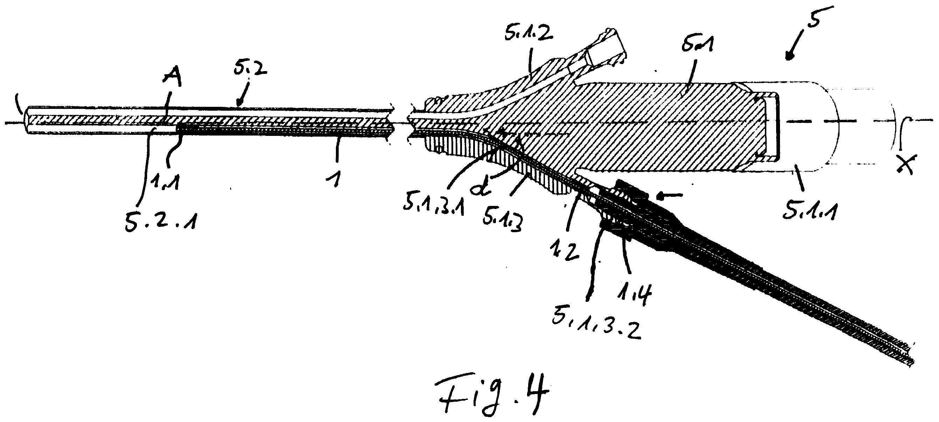

Der Einsatz der erfindungsgemäßen Sensoreinheit bei einem Endoskop ist in der Fig. 4 dargestellt und wird im Folgenden unter Bezug auf diese Figur näher erläutert.

Die dortige Endoskop-Vorrichtung 5 weist im dargestellten Ausführungsbeispiel einen proximalen Einführkopf 5.1 und einen sich von diesem distal erstreckenden Schaft 5.2, mit mindestens einem sich axial im Schaft 5.2 erstreckenden länglichen zylindrischen Hohlraum 5.2.1 auf. Der Einführkopf 5.1 hat hier drei Abzweigungen oder Ansätze 5.1.1 bis 5.1.3, nämlich einen Ansatz 5.1.1 zum Anschluss eines Lichtleiters und der Optik (Kamerasystem) und eines Arbeitskanals zur Einführung von chirurgischen Instrumenten.

Durch einen Einlass 5.1.3.1 des Ansatzes 5.1.3 erstreckt sich eine Sensoreinheit 1 in den axial gestreckten Hohlraum 5.2.1 des Schafts 5.2. Die Sensoreinheit zeigt in der oben beschriebenen Weise mit Abstand in ihrer Längserstreckung zwei Sensoren 1.1 und 1.2 in Form von Spulen. Von den Spulen 1.1, 1.2 erstrecken sich Anschlussdrähte (hier nicht dargestellt) in proximaler Richtung bis zu einem jeweiligen proximalen Anschluss- oder Kontaktende des jeweiligen Drahtes, gegebenenfalls in der Ausbildung eines Steckers zum Anschluss der Drähte an die Steuer- und Verarbeitungselektronik 3 des Navigationssystems 2 (nicht dargestellt).

Die Spule 1.1 findet sich am distalen Ende der Sensoreinheit 1 und damit innerhalb des achsparallel zur Mittelachse X verlaufenden Schaftes 5.2 des Endoskops 5 und ist daher ebenfalls parallel zur Mittelachse X ausgerichtet. Die mit Abstand zur Spule 1.1 an der Sensoreinheit 1 angeordnete Spule 1.2 findet sich in dem unter einem endlichen Winkel zur Mittelachse X verlaufenden Ansatz 5.1.3 des Einführkopfes 5.1., indem der Lumenabschnitt des Hohlraums 5.1.3.1 ebenfalls unter einem endlichen Winkel zur Mittelachse X verläuft, schließt die Ausrichtung oder Erstreckung der Spule 1.2 im Ansatz 5.1.3 ebenfalls einen endlichen Winkel zur Mittelachse X ein. Die beiden Spulen 1.1, 1.2 sind daher nicht parallel zueinander, sondern unter einem endlichen Winkel zueinander ausgerichtet.

Bei einem extern angelegten inhomogenen elektromagnetischen Feld - wie durch den Feldgenerator 2.1 -, in dem sich die Spulen 1.1, 1.2, befinden, nehmen diese daher das Feld unterschiedlich wahr und senden an die Steuer- und Verarbeitungselektronik 3 unterschiedliche Signale. Durch diese unterschiedliche Ausrichtung der Spulen 1.1, 1.2 wird daher die vollständige Platzierung und Orientierung der Endoskop-Vorrichtung im elektromagnetischen Feld und damit im Raum exakt bestimmt.

Die Sensoreinheit 1 weist, wie schon oben gesagt, ein fest angeordnetes Luer-Adapterteil 1.4 auf. Dieses ist mit einem komplementierten Luer-Adapterteil 1.4, 5.1.3.2 am Ansatz 5.1.3 des Endoskops 5 verbindbar - hier verbunden - , die beide zusammen ein Luer-Adapter 1.4, 5.1.3.2 bilden. Der Ansatz 5.1.3 und die Sensoreinheit 1 können so durch den Luer-Adapter in an sich bekannter Weise bajonettartig fest miteinander verbunden werden. Damit ist der Ort des Adapterteils 1.4 relativ zum Ansatz 5.1.3 und auch zum Kopf 5.1 sowie zum Schaft 5.2 der Endoskop-Vorrichtung 5 im befestigten Zustand definiert. Da die Sensoreinheit 1, wie gesagt, axial fest mit dem Halter versehen ist, ist damit auch die Längsposition der Spulen 1.1, 1.2 und insbesondere der distalen Spule 1.1 im Schaft 5.2 und damit der Endoskop-Vorrichtung 5 festgelegt und damit auch der Abstand insbesondere der axiale Abstand der Spule 1.1 zum distalen Ende 5.1.2 des Schaftes 5.2. Damit kann durch das Sensorsignal der Spule 1.1 im angelegten elektromagnetischen Feld auch deren Ort und aufgrund des festen Abstandes zum distalen Ende 5.1.2 des Schaftes 5.2 der Ort des distalen Endes 5.2.1 des Schaftes 5.2 im elektromagnetischen Feld und damit im Raum bestimmt werden.

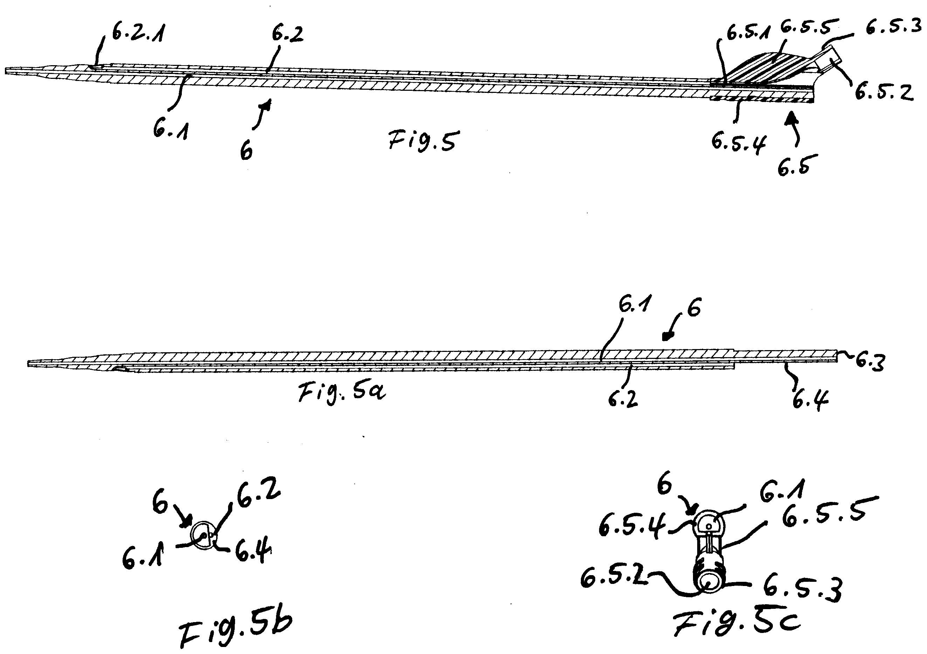

Die Fig. 5 zeigt einen doppelt kanulierten Führungsstab 6 im Längsschnitt mit einem zentralen länglichen, sich Längs durch den gesamten Führungsstab erstreckenden zylindrischen ersten Hohlraum 6.1 und einem parallel zu diesem sich über den größten Teil der Länge des Führungsstabs 6 erstreckenden zweiten zylindrischen Hohlraum 6.2. Dieser mündet distal in einer seitlichen Öffnung 6.2.1. Proximal mündet der Hohlraum 6.2 ebenfalls mit Abstand zur proximalen Stirnseite 6.3 des Führungsstabs 6, während der Hohlraum 6.1 bis zum proximalen Ende 6.3 verläuft. Im proximalen Bereich ist der Führungsstab 6 neben dem Hohlraum 6.1 verjüngt und mit einer achsparallelen Abflachung 6.4 versehen.

Auf das proximale Ende des Führungsstabs 6 ist ein Adapter 6.5 aufsetzbar, der einen den Hohlraum 6.2 fluchtend verlängernden Hohlraum 6.5.1 aufweist, der wiederum in einem Eingangsbereich 6.5.2 des Adapters 6.5 endet. Der Eingangsbereich 6.5.2 weist einen endlichen Winkel zur axialen Erstreckung des Hohlraums 6.2 auf und zwar vorzugsweise einen Winkel von 35° zur Längsachse des Führungsstabs bzw. der Erstreckungsrichtung des Hohlraums 6.2.

Zur Stabilisierung des Adapters 6.5 ist im Winkel zwischen seinem Steckteil 6.5.4 und dem Eingangsbereich 6.5.2 eine Verbindungsplatte 6.5.5 vorgesehen. Der Adapter 6.5 ist einteilig ausgebildet und besteht vorzugsweise aus Kunststoff.

Durch den Eingangsbereich 6.5.2 wird bis in den Hohlraum 6.2 eine Sensoreinheit gemäß der Fig. 1, 2 eingeschoben, wobei sich der distale Sensor 1.1 der Sensoreinheit 1 im distalen Bereich des Hohlraums 6.2 befindet und der Sensor 1.2 bei voll aufgestecktem Adapter 1.4 mit dessen zylindermantelförmigem distalen Ringraum 1.8 auf der Zylindermantelwandung 6.5.3 des Adapters 6.5 im Bereich des abgewinkelten Eingangsbereichs 6.5.2.

Die unterschiedliche Ausrichtung der beiden Sensoren 1.1, 1.2 der im Führungsstab 6 eingesteckten Sensoreinheit 1 und insbesondere der genaue Winkel der Sensoren 1.1, 1.2 zueinander kann durch eine Auswerteeinheit detektiert und hierauf die Anzeige auf den Bildschirm einer Steuereinheit entsprechend ausgewählt bzw. angepasst werden, wie dies weiter hinten im Text sowohl für eine Hohlnadel als auch für ein Endoskop im Detail beschrieben ist.

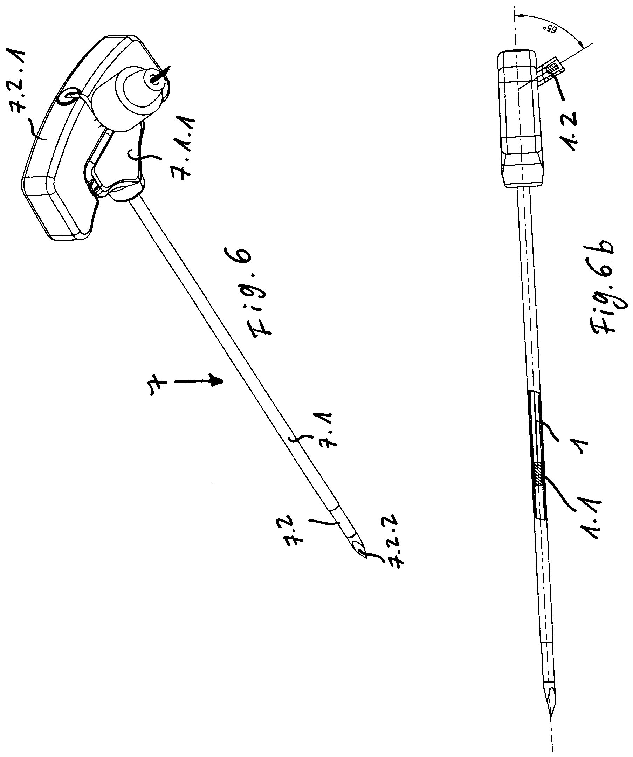

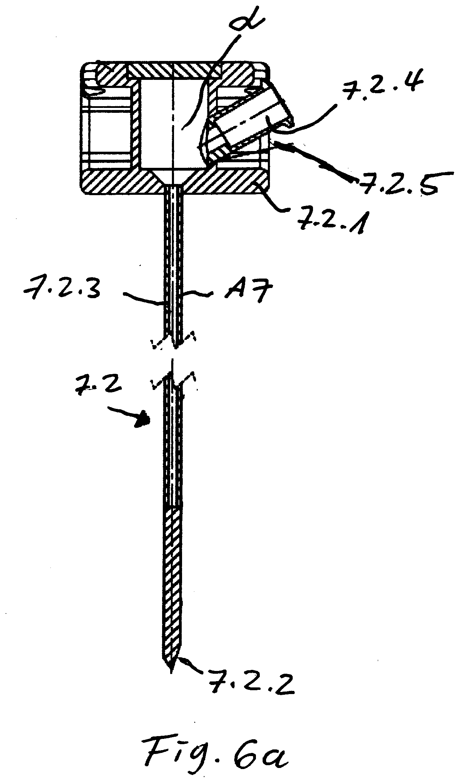

Als weiteres im Rahmen des Erfindungsgegenstandes und insbesondere des Detektionssystems detektierbaren chirurgischen Instruments ist eine Punktionsnadel 7 nach Yamshidi vorgesehen, wie sie in den Fig. 6 dargestellt ist. Eine derartige Punktionsnadel 7 ist zweiteilig ausgebildet. Sie weist zum einen eine Kanüle 7.1 und zum anderen ein Stilett 7.2, die jeweils mit einem Griffteil 7.1.1 und 7.2.1 versehen sind. Das Stilett 7.2 überragt die Kanüle 7.1 und weist ein spitzes distales Ende 7.2.2 auf, das konisch oder als Trokar ausgebildet sein soll. Bei üblichen derartigen Punktionsnadeln 7 ist das Stilett massiv ausgebildet.

Das erfindungsgemäße Stilett 7.2. weist einen axialen länglichen zylindrischen Hohlraum 7.2.3 auf, wie er im Längsschnitt des Stiletts 7.2 der Fig. 6a erkennbar ist. Dieser erstreckt sich vom proximalen Adapterteil 7.2.1 des Stiletts 7.2.1 durch das Stilett 7.2 und endet mit einem Abstand zur distalen Spitze 7.2.2. Das Adapterteil 7.2.1 weist wiederum ein Eingangsbereich 7.2.4 mit einer im Wesentlichen zylindrischen Mantelwandung 7.2.5 auf, der in einem endlichen Winkel zur Erstreckungsrichtung des Stiletts 7.2 bzw. dessen Achse A7 ausgerichtet ist. Der Relativwinkel α des Eingangsbereichs 7.2.4 zur Achse A7 des Stiletts weicht von den Relativwinkeln anderer im Rahmen des erfindungsgemäßen Detektorsystems einsetzbarer chirurgischer Instrumente ab und zwar mindestens um 5° und beträgt im dargestellten Ausführungsbeispiel 65°.

Auch durch den Eingangsbereich 7.2.4 des Stiletts 7.2 der Punktionsnadel 7 wird eine Sensoreinheit 1 der Fig. 1, 2 bis in den axialen Hohlraum 7.2.3 eingeschoben und das Adapterteil 1.4 der Sensoreinheit 1 mit seinem zylindrischen Ringraum 1.8 am Mantel 7.2.5 des Eingangsbereichs 7.2.4 so positioniert, dass der Sensor 1 im distalen Endbereich des axialen Hohlraums 7.2.3 des Stiletts 7.2 zu liegen kommt, der Sensor 1.2 aber im hierzu unter dem genannten endlichen Winkel ausgerichteten Einlass 7.2.5.

Derart kann die Relativposition der Sensoren 1.1 und 1.2 in einem elektromagnetischen Feld und insbesondere die genaue Winkelausrichtung durch die Auswerteeinheit wiederum bestimmt werden und damit als chirurgisches Instrument die Punktionsnadel 7 nach Yamshidi identifiziert werden und entsprechend die Anzeige auf dem Monitor der Verarbeitungselektronik angezeigt werden, wie dies, wie schon gesagt, im Folgenden im Einzelnen für eine - gestreckte - Hohlnadel und ein Endoskop beschrieben ist. Die dortigen Ausführungen treffen in entsprechender Weise ebenso wie für den oben beschriebenen Führungsstab als auch für die hier beschriebene Punktionsnadel nach Yamshidi zu.

Ein erfindungsgemäßes Detektionssystem wird insbesondere durch die Sensoreinheit 1, mindestens zwei der Instrumente 2.2, 5, 6, 7 und eine Auswerteeinheit 3.1.3 gebildet, die im Folgenden im Rahmen einer umfassenden Steuer- und Verarbeitungselektronik 3 beschrieben wird.

Die Fig. 7 zeigt eine schematische blockbildartige Darstellung eines erfindungsgemäßen intraoperativen Navigationssystems 2 und seine wesentlichen Komponenten. Teile des Navigationssystems 2 sind die Sensoreinheit 1, die Steuer- und Verarbeitungselektronik 3, der Feldgenerator 2.1, mindestens ein rotationssymmetrisches Instrument 2.2, wie einer Hohlnadel 2.2 und mindestens ein Endoskop 2.3.

Die Steuer- und Verarbeitungselektronik 3 weist eine Navigationseinheit 3.1 und ein Kamerasystem 3.2 für das Endoskop 2.3 auf, wie dies in der Fig. 7 dargestellt ist.

Die Navigationseinheit 3.1 weist zunächst eine Steuereinheit 3.1.1 zur Ansteuerung des Feldgenerators 2.1 auf. Weiterhin weist sie eine mit der Sensoreinheit 1 verbundene Empfangseinheit 3.1.2 zum Empfang von Signalen S1, S2 der Sensoreinheit und zur Weiterverarbeitung derselben auf. Die so empfangenen und angepassten Sensorsignale werden durch eine Auswerteeinheit 3.1.3 weiterverarbeitet, die durch Softwaremodule realisiert sein kann. Die Auswerteeinheit 3.1.3 weist zunächst ein Funktionsmodul 3.1.3.3.1zur Instrumentenerkennung auf, also ob ein rotationssymmetrisches Instrument 2.2, wie eine Hohlnadel, oder ein nichtrotationssymmetrisches Instrument, wie ein Endoskop 2.3 angeschlossen sind.

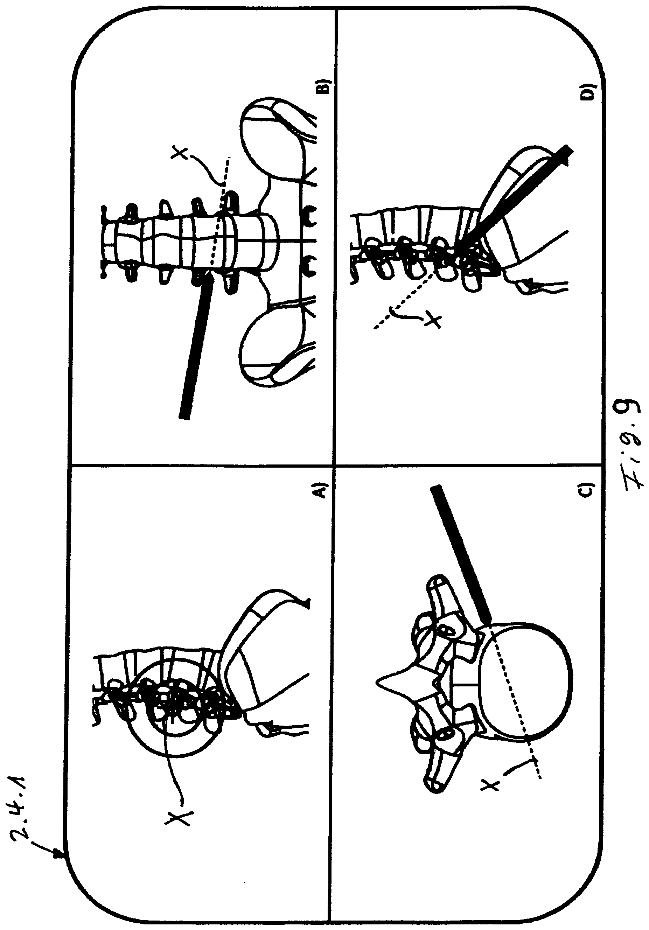

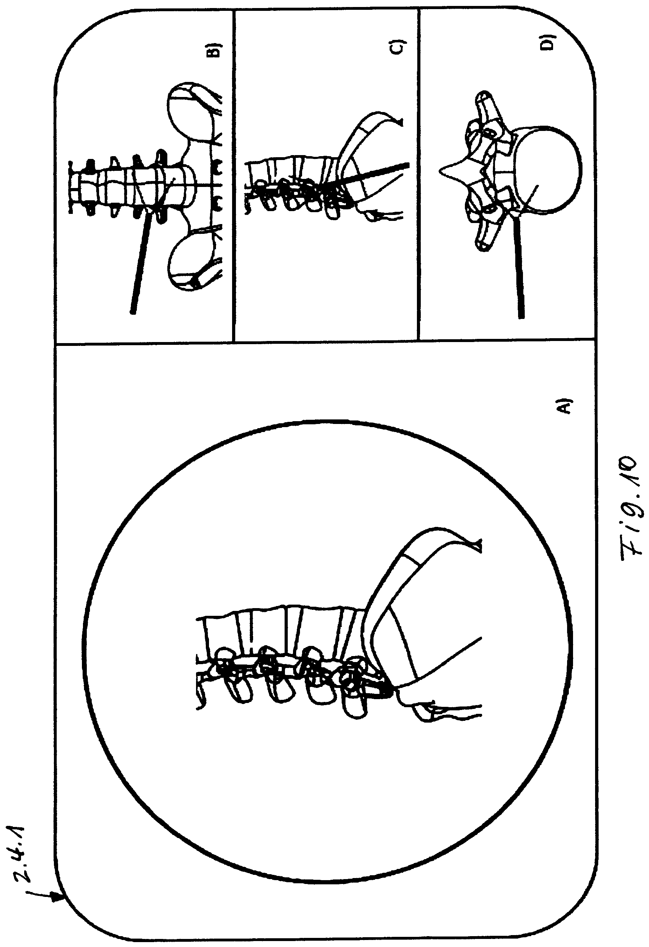

Das Funktionsmodul 3.1.3.3.1 wiederum ist mit einer Layout-Steuerung 3.1.3.3 verbunden, mittels derer das Layout der Darstellung auf dem Monitor gesteuert wird und zwar über das Funktionsmodul 3.1.3.3.1 bei rotationssymmetrischem Instrument 2.2 entsprechend der Fig. 9 und über das Funktionsmodul 3.1.3.3.1 bei einem Endoskop 2.3 entsprechend der Fig. 10 unter Heranziehung des Endoskop-Bildes.

Die Auswerteeinheit 3.1.3 weist weiter ein Einspeisemodul 3.1.3.2 zur Einspeisung des Videosignals des Endoskops 2.3 in die durch das Funktionsmodul 3.1.3.1 bewirkte Darstellung des Monitorbildes der Fig. 10 auf.

Das Videosignal des Endoskops 2.3 wird der Navigationseinheit 3.1 über ein Kamerasystem 3.2 des Endoskops 2.3 zugeführt, das einerseits eine Auswerteeinheit 3.1.3 zur Verarbeitung des Bildes des Endoskops 2.3 und andererseits eine Steuereinheit 3.1.1 zur Steuerung der Kamera des Endoskops aufweist.

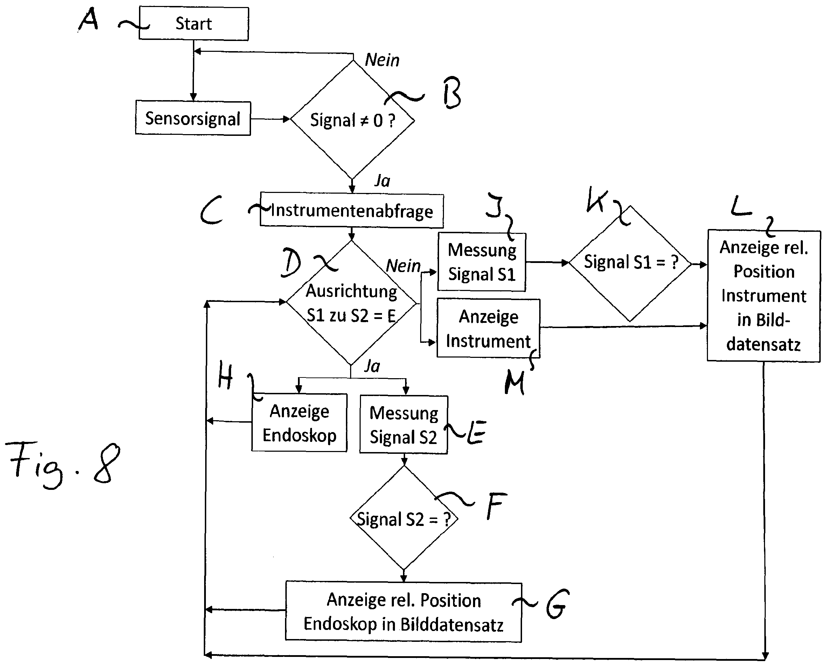

Der Arbeitsablauf des erfindungsgemäßen Navigationssystems ist entsprechend der Fig. 8 folgender:
Nach Starten der Navigationseinheit 3.1 werden die Signale der Sensorik abgefragt. Sobald der Anwender die Sensoreinheit 1 in das Arbeitsfeld des Feldgenerators 2.1 bringt, kann ein Signal erkannt und gemessen werden. Ist gemäß Abfolge B das Sensorsignal ungleich dem Wert Null und entsprechend der Sensoreinheit 1, erfolgt eine Instrumentenabfrage C. Hierfür wird die Ausrichtung der beiden Sensorspulen (Schritt D) ausgewertet. Sind dabei die beiden Spulen 1.1, 1.2 zueinander entsprechend der Geometrie des Spülkanals unter einem endlichen Relativwinkel ungleich Null (Abstand und Winkel), wird dies von der Software erkannt und entsprechend das Endoskop 2.3 als navigiertes Instrument angezeigt. Des Weiteren wird eine Layout-Anpassung gemäß der Fig. 10 vorgenommen. Wurde das Endoskop 2.3 als navigiertes Instrument erkannt, wird ein Signal der proximalen Sensorspule 1.2 genutzt um die Position des Endoskops im Arbeitsfeld zu messen (Schritt E) und aufgrund der Messung F die Position relativ zum 3D Bilddatensatz anzuzeigen (Schritt G). Weiter wird, wie schon beschrieben, das Endoskop-Bild im Monitor eingeführt. Insbesondere kann aufgrund der bekannten Lage der Spule 1.2 im Endoskop auf die Position des distalen Endes des Endoskops im Raum geschlossen werden und diese im Bild des Wirbelsäulenbereichs dargestellt werden, wie dies in Fig. 10 dargestellt ist.

Wird in Schritt D eine Ausrichtung der beiden Sensorspulen 1.1, 1.2 ungleich der genau definierten Lage entsprechend der eines Spülkanals des Endoskops zu dessen Hauptachse von in der Regel 25° bestimmt, wird ein rotationssymmetrisches Instrument (Nadel) erkannt und zusammen mit der entsprechenden Layout-Darstellung von dem Navigationssystem ausgegeben (Schritt H). Die Sensorspule 1.1 wird zur Bestimmung der Instrumentenlage im Arbeitsfeld genutzt (Schritt J) und entsprechend der gemessenen Lage (Schritt K) im 3D Bilddatensatz angezeigt (Schritt L).

Erkennt das Navigationssystem aufgrund der axialen Ausrichtung der beiden Spulen 1.1, 1.2 im inhomogenen Feld, also einer Ausrichtung unter einem Relativwinkel, der nicht der Anordnung des abgewandten Anschlusses eines Endoskops zu dessen Hauptachse in der Regel von 25° entspricht, insbesondere unter diesem Winkel liegt, dass eine Hohlnadel 2.2 als rotationssymmetrisches Instrument im Einsatz ist, so wird dem Anwender automatisch auf einem Monitor 2.4 eine Darstellung angezeigt, in der der Körper des Patienten, genauer eines Wirbelsäulenbereichs bzw. Teile des Bereichs, - hier der Wirbelsäule 4 - aus vier Sichten unter Überlagerung der Darstellung des Operationsinstruments bzw. der Hohlnadel 2.2 dargestellt werden. Die Darstellungen beruhen auf einem Bilddatensatz eines Patienten, der mittels einer Computertomografie (CT) oder einer Magnetresonanztomographie (MRT) gewonnen wird (aufgrund von Patentformerfordernissen zeigen die Fig. 9 ebenso wie die Fig. 10 lediglich vereinfachte schematische zeichnerische Darstellungen des tatsächlich auf dem Monitor 2.4 dargestellten farbigen oder Graustufen-Bildes und ebenfalls lediglich in schwarzer Darstellung das Operationsinstrument bzw. den Schaft und das distale Ende des Endoskops, welches tatsächlich auf dem Monitor 2.4 abgesetzt farbig dargestellt wird).

Im Monitorbild 2.4.1 wird links oben (A) der Bereich der Wirbelsäule 4 entsprechend einer Zielanzeige des Instruments dargestellt, d.h. in Achsrichtung des Instruments bzw. der Hohlnadel 2.2 in Richtung der Achse X des Instruments bzw. der Hohlnadel 2.2 (hierzu auch die weiteren Darstellungen der Fig. 9). Rechts daneben (B) erfolgt eine sagittale Anzeige, also die Darstellung einer Ebene, deren Senkrechte sagittal gerichtet ist, also vom Rücken zum Brustkorb des Patienten. Die Darstellung links unten (C) ist eine axiale Anzeige, also entsprechend der Erstreckungsrichtung der Wirbelsäule. Die letzte Darstellung rechts unten (D) zeigt eine koronale Anzeige, also von der Seite her.

Erkennt das Navigationssystem 2 ein Endoskop durch Ausrichtung der beiden Spulen 1.1, 1.2 unter einem endlichen Winkel entsprechend dem Winkel zwischen Hauptachse des Endoskops und proximalen Abzweigung von in der Regel 25°, so wird im großen Bildteil links des Monitors entsprechend der Fig. 10 das echte Bild der Kamera des Endoskops dargestellt. Die Abbildungen rechts von oben nach unten (B-D) zeigen wieder die unter Bezug auf Fig. 9 beschriebenen Anzeigen und zwar hier B sagittal, C koronal und D axial, wie auch ein Vergleich mit der Fig. 9 zeigt.

Das erfindungsgemäße Navigationssystem erkennt aufgrund der Ausrichtung der beiden Spulen der Sensoreinheit zueinander unmittelbar die Art des chirurgischen Instruments, dessen distales Ende sich am Operationsort befindet und dem Operateur wird automatisch die entsprechende für ihn relevante Darstellung auf dem Monitor 2.4 angezeigt.

Damit erkennt ein Operateur aufgrund der Auswertung der Sensorsignale und einer Bildanzeige auf einem Bildschirm der Auswerteeinrichtung genau die Position des distalen Endes 5.2.1 des Schaftes 5.2 und damit auch der Endoskop-Vorrichtung 5 und weiß damit wo er exakt mit seinen Instrumenten, die er gegebenenfalls durch anderen Lumen der Endoskop-Vorrichtung ein und durch diese hindurchführt, arbeitet. Entsprechendes gilt bei der Ausrichtung der Sensoreinheit 1 in eine Hohlnadel 2.2.

## Patentansprüche

1. Detektionssystem zur automatischen Detektion von chirurgischen Instrumenten
- mit mindestens zwei chirurgischen Instrumenten, die jeweils mindestens einen sich längs einer Längsachse des Instruments (2.2, 5, 6, 7) erstreckenden Hohlraum (5.2.1, 6.2, 7.2.3) und einen proximalen Eingangsbereich (5.1, 3.1, 6.5.2, 7.2.4) zu dem Hohlraum aufweisen, wobei ein Winkel des proximalen Eingangsbereichs zur Längserstreckung des Hohlraums bei den mindestens zwei chirurgischen Instrumenten unterschiedlich ist,
- mit einer zwei elektromagnetische Sensoren (1.1, 1.2) aufweisenden in den Hohlraum (5.2.1, 6.2, 7.2.3) einführbaren Sensoreinheit (1), dessen einer sich axial erstreckend im Hohlraum (5.2.1, 6.2, 7.2.3), dessen anderer im Eingangsbereich (5.1, 3.1, 6.5.2, 7.2.4) angeordnet ist,
- mit einem Feldgenerator (2.1) zur Erzeugung eines elektromagnetischen Feldes, und
- mit einer Auswerteeinheit (3.1.3) zur Auswertung von den elektromagnetischen Sensoren (1.1, 1.2) entsprechend ihrer Lage im Feld des Feldgenerators (2.1) an die Auswerteeinheit (3.1.3) übermittelter Signale und zur Bestimmung der genauen Winkelausrichtung der Sensoren (1.1, 1.2)in dem elektromagnetischen Feld um die Art des chirurgischen Instruments zu identifizieren.

2. System nach Anspruch 1, **gekennzeichnet durch** mindestens zwei der folgenden chirurgischen Instrumente: Hohlnadel (2.2), Führungsstab (6), Zugangsnadel (6) nach Yamshidi und/oder Endoskop (5).

3. System nach Anspruch 2, **dadurch gekennzeichnet, dass** der Führungsstab (6) doppelt kanuliert ist.

4. System nach Anspruch 1 oder 2, **gekennzeichnet durch** mindestens vier unterschiedliche chirurgische Instrumente.

5. System nach Anspruch 4, **dadurch gekennzeichnet, dass** die Winkel der Eingangsbereiche (5.1, 3.1, 6.5.2, 7.2.4) zur Längserstreckung des Hohlraums (5.2.1, 6.2, 7.2.3) zweier chirurgischer Instrumente sich um mindestens 5° unterscheiden.

6. System nach Anspruch 5, **dadurch gekennzeichnet, dass** die Eingangsbereiche (5.1, 3.1, 6.5.2, 7.2.4) Winkel von 0°, 15°, 25°, 35°, 45°, 55°, 65° oder 75° zur Längserstreckung des Hohlraums (5.2.1, 6.2, 7.2.3) aufweisen.

7. System nach Anspruch 6, **dadurch gekennzeichnet, dass** die elektromagnetischen Sensoren (1.1, 1.2) einen festen Längsabstand zueinander aufweisen.

8. System nach Anspruch 7, **dadurch gekennzeichnet, dass** die elektromagnetischen Sensoren (1.1, 1.2) Spulen aus elektrisch leitfähigem Draht sind.

9. System nach Anspruch 8, **dadurch gekennzeichnet, dass** die Spulen aus Metall, insbesondere Kupfer oder Silber, bestehen.

10. System nach Anspruch 9, **dadurch gekennzeichnet, dass** die Sensoreinheit (1) einen die elektromagnetischen Sensoren (1.1, 1.2) ummantelnden flexiblen Schutzschlauch (1.3) aufweist.

11. System nach Anspruch 10, **dadurch gekennzeichnet, dass** der Schutzschlauch aus Kunststoff, insbesondere aus biokompatiblem, sterilisierbarem Kunststoff, wie Polyether besteht.

12. System nach einem der vorangehenden Ansprüche, **dadurch gekennzeichnet, dass** der proximale Eingangsbereich (5.1, 3.1, 6.5.2, 7.2.4) eines Luer-Adapters ein Adapterteil, vorzugsweise eines chirurgischen Instruments (2.2, 5, 6, 7), aufweist.

13. System nach einem der vorangehenden Ansprüche, **gekennzeichnet durch** eine elektrische Verbindungsleitung (1.5) der elektromagnetischen Sensoren (1.1, 1.2) am proximalen Ende, insbesondere zur Verbindung mit der Auswerteeinheit (3.1.3).

14. System nach Anspruch 13, **dadurch gekennzeichnet, dass** die elektrische Verbindungsleitung (1.5) von einem Schutzschlauch (1.3) aus biokompatiblem, sterilisierbarem Kunststoff, insbesondere Silikon, umgeben ist.

15. System nach Anspruch 14, **dadurch gekennzeichnet, dass** der Schutzschlauch (1.3) unmittelbar proximal des Adapterteils (1.4) der Sensoreinheit (1) von einem Knickschutz (1.7) umgeben ist.

16. System nach Anspruch 15, **dadurch gekennzeichnet, dass** der Knickschutz (1.7) aus biokompatiblem, sterilisierbarem Kunststoff, insbesondere Silikon, umgeben ist.

17. System nach einem der vorangehenden Ansprüche, **dadurch gekennzeichnet, dass** die Auswerteeinheit (3.1.3) Teil einer Steuer- und Verarbeitungselektronik (3.1) ist.

18. System nach einem der vorangehenden Ansprüche, **gekennzeichnet durch** einen Monitor (2.4).

19. System nach einem der vorangehenden Ansprüche, **gekennzeichnet durch** Ausbildung zur Darstellung mindestens zweier chirurgischer Instrumente, wie Hohlnadel (2.2), eines Führungsstabs (6), der insbesondere doppelt kanuliert ist, einer Zugangsnadel (7) und/oder Endoskop (5), insbesondere des distalen Endes (5.1.2, 5.2.1), chirurgische Instrumente überlagert in einem Bild eines Wirbelsäulenbereichs eines Patienten.

20. Intraoperative Navigationsvorrichtung (2) mit einer Steuer- und Verarbeitungselektronik (3) und einem Detektionssystem nach einem der vorangehenden Ansprüche, **dadurch gekennzeichnet, dass** die Steuer- und Verarbeitungselektronik (3) eine Navigationseinheit (3.1), in die die Auswerteeinheit (3.1.3) angepasst ist, aufweist.

## Claims

1. Detection system for automatically detecting surgical instruments
- having at least two surgical instruments, which each comprise at least one cavity (5.2.1, 6.2, 7.2.3) extending along a longitudinal axis of the instrument (2.2, 5, 6, 7) and a proximal entry region (5.1, 3.1, 6.5.2, 7.2.4) to the cavity, wherein an angle of the proximal entry region in relation to the longitudinal extension of the cavity is different in the at least two surgical instruments,
- having a sensor unit (1), which comprises two electromagnetic sensors (1.1, 1.2), is insertable into the cavity (5.2.1, 6.2, 7.2.3), and one sensor of which is arranged axially extending in the cavity (5.2.1, 6.2, 7.2.3), and the other sensor of which is arranged in the entry region (5.1, 3.1, 6.5.2, 7.2.4),
- having a field generator (2.1) for generating an electromagnetic field, and
- having an analysis unit (3.1.3) for analyzing signals transmitted by the electromagnetic sensors (1.1, 1.2) to the analysis unit (3.1.3) in accordance with the location thereof in the field of the field generator (2.1) and for detecting the precise angular orientation of the sensors (1.1, 2.1) in the electromagnetic field to identify the type of the surgical instrument.

2. System according to claim 1, **characterized by** at least two of the following surgical instruments: hollow needle (2.2), guide rod (6), penetration needle (6) according to Jamshidi, and/or endoscope (5).

3. System according to claim 2, **characterized in that** the guide rod (6) is doubly cannulated.

4. System according to claim 1 or 2, **characterized by** at least four different surgical instruments.

5. System according to claim 4, **characterized in that** the angles of the entry regions (5.1, 3.1, 6.5.2, 7.2.4) in relation to the longitudinal extension of the cavity (5.2.1, 6.2, 7.2.3) of two surgical instruments differ by at least 5°.

6. System according to claim 5, **characterized in that** the entry regions (5.1, 3.1, 6.5.2, 7.2.4) have angles of 0°, 15°, 25°, 35°, 45°, 55°, 65°, or 75° in relation to the longitudinal extension of the cavity (5.2.1, 6.2, 7.2.3) .

7. System according to claim 6, **characterized in that** the electromagnetic sensors (1.1, 1.2) have a fixed longitudinal spacing in relation to one another.

8. System according to claim 7, **characterized in that** the electromagnetic sensors (1.1, 1.2) are coils made of electrically conductive wire.

9. System according to claim 8, **characterized in that** the coils consist of metal, in particular copper or silver.

10. System according to claim 9, **characterized in that** the sensor unit (1) comprises a flexible protective tubing (1.3) jacketing the electromagnetic sensors (1.1, 1.2).

11. System according to claim 10, **characterized in that** the protective tubing consists of plastic, in particular of biocompatible plastic which can be sterilized, such as polyether.

12. System according to one of the preceeding claims, **characterized in that** the proximal entry region (5.1, 3.1, 6.5.2, 7.2.4) of a Luer adapter comprises an adapter part, preferably of a surgical instrument (2.2, 5, 6, 7).

13. System according to one of the preceeding claims, **characterized by** an electrical connecting line (1.5) of the electromagnetic sensors (1.1, 1.2) at the proximal end, in particular for connection to the analysis unit (3.1.3).

14. System according to claim 13, **characterized in that** the electrical connecting line (1.5) is enclosed by a protective tubing (1.3) made of biocompatible plastic which can be sterilized, in particular silicone.

15. System according to claim 14, **characterized in that** the protective tubing (1.3) is enclosed directly proximal of the adapter part (1.4) of the sensor unit (1) by a buckling safeguard (1.7).

16. System according to claim 15, **characterized in that** the buckling safeguard (1.7) is enclosed by biocompatible plastic which can be sterilized, in particular silicone.

17. System according to one of the preceeding claims, **characterized in that** the analysis unit (3.1.3) is part of a control and processing electronics unit (3.1).

18. System according to one of the preceeding claims, **characterized by** a monitor (2.4).

19. System according to one of the preceeding claims, **characterized by** design to represent at least two surgical instruments, such as hollow needles (2.2), a guide rod (6), which is in particular doubly cannulated, a penetration needle (7), and/or an endoscope (5), in particular of the distal end (5.1.2, 5.2.1), surgical instruments overlaid in an image of a spinal column region of a patient.

20. Intraoperative navigation device (2) having a control and processing electronics unit (3) and a detection system according to one of the preceding claims, **characterized in that** the control and processing electronics unit (3) comprises a navigation unit (3.1), in which the analysis unit (3.1.3) is adapted.

## Revendications

1. Système de détection destiné à détecter automatiquement des instruments chirurgicaux
- comportant au moins deux instruments chirurgicaux, qui présentent respectivement au moins un espace creux (5.2.1, 6.2, 7.2.3) s'étendant le long d'un axe longitudinal de l'instrument (2.2, 5, 6, 7) et une zone d'entrée proximale (5.1, 3.1, 6.5.2, 7.2.4) par rapport à l'espace creux, un angle de la zone d'entrée proximale par rapport à l'étendue longitudinale de l'espace creux pour les au moins deux instruments chirurgicaux étant différent,
- comportant une unité de détecteur (1) présentant deux capteurs électromagnétiques (1.1, 1.2) et pouvant être introduite dans l'espace creux (5.2.1, 6.2, 7.2.3), l'un des capteurs étant agencé dans l'espace creux (5.2.1, 6.2, 7.2.3) en s'étendant axialement et l'autre étant agencé dans la zone d'entrée (5.1, 3.1, 6.5.2, 7.2.4),
- comportant un générateur de champ (2.1) destiné à générer un champ électromagnétique et
- comportant une unité d'évaluation (3.1.3) destinée à évaluer des signaux transmis par les capteurs électromagnétiques (1.1, 1.2) en fonction de leur position dans le champ du générateur de champ (2.1) à l'unité d'évaluation (3.1.3) et à déterminer l'orientation angulaire exacte des capteurs (1.1, 1.2) dans le champ électromagnétique pour identifier le type de l'instrument chirurgical.

2. Système selon la revendication 1, **caractérisé par** au moins deux des instruments chirurgicaux suivants : aiguille creuse (2.2), tige de guidage (6), aiguille d'accès (6) selon Yamshidi et/ou endoscope (5).

3. Système selon la revendication 2, **caractérisé en ce que** la tige de guidage (6) est à double canule.

4. Système selon la revendication 1 ou 2, **caractérisé par** au moins quatre instruments chirurgicaux différents.

5. Système selon la revendication 4, **caractérisé en ce que** les angles des zones d'entrée (5.1, 3.1, 6.5.2, 7.2.4) par rapport à l'étendue longitudinale de l'espace creux (5.2.1, 6.2, 7.2.3) de deux instruments chirurgicaux diffèrent d'au moins 5°.

6. Système selon la revendication 5, **caractérisé en ce que** les zones d'entrée (5.1, 3.1, 6.5.2, 7.2.4) présentent des angles de 0°, 15°, 25°, 35°, 45°, 55°, 65° ou 75° par rapport à l'étendue longitudinale de l'espace creux (5.2.1, 6.2, 7.2.3).

7. Système selon la revendication 6, **caractérisé en ce que** les capteurs électromagnétiques (1.1, 1.2) présentent une distance longitudinale fixe l'un par rapport à l'autre.

8. Système selon la revendication 7, **caractérisé en ce que** les capteurs électromagnétiques (1.1, 1.2) sont des bobines en fil électriquement conducteur.

9. Système selon la revendication 8, **caractérisé en ce que** les bobines sont en métal, en particulier en cuivre ou en argent.

10. Système selon la revendication 9, **caractérisé en ce que** l'unité de capteur (1) présente un tuyau de protection (1.3) souple entourant les capteurs électromagnétiques (1.1, 1.2).

11. Système selon la revendication 10, **caractérisé en ce que** le tuyau de protection est en matériau synthétique, en particulier en matériau synthétique biocompatible, stérilisable, tel que le polyéther.

12. Système selon l'une des revendications précédentes, **caractérisé en ce que** la zone d'entrée proximale (5.1, 3.1, 6.5.2, 7.2.4) d'un embout Luer présente une partie d'adaptateur, de préférence d'un instrument chirurgical (2.2, 5, 6, 7).

13. Système selon l'une des revendications précédentes, **caractérisé par** une ligne de connexion électrique (1.5) des capteurs électromagnétiques (1.1, 1.2) au niveau de l'extrémité proximale, en particulier pour la connexion avec l'unité d'évaluation (3.1.3).

14. Système selon la revendication 13, **caractérisé en ce que** la ligne de connexion électrique (1.5) est entourée par un tuyau de protection (1.3) en matériau synthétique biocompatible, stérilisable, en particulier en silicone.

15. Système selon la revendication 14, **caractérisé en ce que** le tuyau de protection (1.3) est entouré d'une protection anti-pliage (1.7) de manière immédiatement proximale par rapport à la partie d'adaptateur (1.4) de l'unité de capteur (1).

16. Système selon la revendication 15, **caractérisé en ce que** la protection anti-pliage (1.7) en matériau synthétique biocompatible, stérilisable, en particulier en silicone, est entourée.

17. Système selon l'une des revendications précédentes, **caractérisé en ce que** l'unité d'évaluation (3.1.3) fait partie d'une électronique de commande et de traitement (3.1).

18. Système, selon l'une des revendications précédentes, **caractérisé par** un moniteur (2.4).

19. Système selon l'une des revendications précédentes, **caractérisé par** un mode de réalisation destiné à représenter au moins deux instruments chirurgicaux, tels qu'une aiguille creuse (2.2), une tige de guidage (6), qui est en particulier à double canule, une aiguille d'accès (7) et/ou un endoscope (5), en particulier l'extrémité distale (5.1.2, 5.2.1), d'instruments chirurgicaux de manière superposée dans une image d'une zone de la colonne vertébrale d'un patient.

20. Dispositif de navigation peropératoire (2) comportant une électronique de commande et de traitement (3) et un système de détection selon l'une des revendications précédentes, **caractérisé en ce que** l'électronique de commande et de traitement (3) présente une unité de navigation (3.1), dans laquelle l'unité d'évaluation (3.1.3) est adaptée.
